# EUROPÄISCHE PATENTSCHRIFT

(11) **EP 1 038 574 B1**
(45) Veröffentlichungstag und Bekanntmachung des Hinweises auf die Patenterteilung: **21.05.2003**
(21) Anmeldenummer: 00114070.6
(22) Anmeldetag: 19.12.1995
(51) Int. Cl.: B01J 13/00

(54) **Verfahren und Vorrichtung zur Herstellung wirkstoffhaltiger Dispersionen**
Method and apparatus for preparing dispersions containing active substances
Procédé et dispositif de fabrication de dispersions contenant des substances actives

(30) Priorität: 19.12.1994 DE 4445341
(43) Veröffentlichungstag der Anmeldung: 27.09.2000
(62) Teilanmeldung aus: 95120019.5
(73) Patentinhaber: BASF AKTIENGESELLSCHAFT, 67056 Ludwigshafen (DE)
(72) Erfinder: Riede, Thomas Dr., 64625 Bensheim (DE); Göbel, Werner, 67149 Meckenheim (DE); Lockemann, Christian Dr., 68163 Mannheim (DE)
(74) Vertreter: Isenbruck, Günter, Dr.

(56) Entgegenhaltungen:
- WO-A-93/17665

## Beschreibung

Die Erfindung bezieht sich auf ein Verfahren und eine Vorrichtung zur Herstellung wirkstoffhaltiger Dispersionen.

In vielen Bereichen der chemischen Industrie müssen heute wirkstoffhaltige Dispersionen hergestellt werden, z. B. in der Farben- und in der Pharmaindustrie. Dabei ist es oft nicht möglich, den Wirkstoff, z. B. den Farbstoff oder den Pharmawirkstoff, unmittelbar in einer wäßrigen Flüssigkeit zu dispergieren. Insbesondere liegen die Wirkstoffpartikel dann nicht in der gewünschten mikronisierten Form vor.

Die Herstellung von feinstverteilten, mikronisierten Carotinoiden in einer wäßrigen Dispersion wird von der deutschen Offenlegungssschrift 29 43 267 beschrieben. Dort wird ein in einem Autoklaven befindliches Carotinoid in einem überkritischen Gas gelöst und die erhaltene Lösung in einer geeigneten wäßrigen kolloidalen Matrix, die sich in einem zweiten Autoklaven befindet, mittels eines Rührorgans dispergiert. Die so erhaltene Dispersion besteht aus wirkstoffhaltigen Tröpfchen von fluidem Gas in Wasser. Dieses zweiphasige System wird entspannt, wobei das Gas kontinuierlich aus dem Autoklaven abgezogen wird, die Flüssigkeit hingegen nur diskontinuierlich nach Beendigung des Prozesses entnommen werden kann. Durch dieses Verfahren wird eine mittlere Teilchengröße des Carotinoids von weniger als 1 Mikron erreicht. Nachteilig ist, daß es während des Entspannungsvorgangs zur Entmischung der Dispersion kommen kann, so daß der Wirkstoff nicht in ausreichender Weise von der flüssigen Matrix stabilisiert wird. Außerdem ist ein Wirkstoffverlust hinzunehmen.

Das europäische Patent EP 0 322 687 schlägt ein Verfahren zur Herstellung einer Arzneiform vor, die einen Wirkstoff und einen Träger umfaßt. Dabei werden ein fluides Gas, ein Wirkstoff und Trägermaterialien, die auch in einer Flüssigkeit gelöst sein können, in einen Sprühturm eingebracht, so daß das fluide Gas Wirkstoff und Träger aufnimmt. In einem Abscheider wird anschließend das fluide Gas von der entstandenen Wirkstoff/Träger-Kombination getrennt, so daß diese aus dem Separator entnommen werden kann. Bei diesem Verfahren werden trockene Wirkstoffpartikel erzeugt, nicht jedoch flüssige Dispersionen. Unter "fluidem Gas" wird eine bei Atmosphärendruck als Gas oder Dampf vorliegende Substanz verstanden, die bis in die Nähe ihres kritischen Punkts oder darüber hinaus verdichtet ist und daher als unteroder überkritisches Fluid vorliegt.

Aufgabe der Erfindung ist die Bereitstellung eines Verfahrens und einer Vorrichtung zur Herstellung wirkstoffhaltiger Dispersionen, die zu einer besonders feinen Verteilung des Wirkstoffes in der Dispersion führen. Insbesondere sollen die mikronisierten Wirkstoffe nicht agglomerieren.

Die Aufgabe wird durch ein Verfahren gelöst, bei dem ein Wirkstoff in einem fluiden Gas aufgelöst wird und das wirkstoffbeladene fluide Gas in einer mit diesem Gas gesättigten Flüssigkeit dispergiert wird, so daß sich eine erste Dispersion aus Flüssigkeit und wirkstoffbeladenem fluidem Gas ausbildet. Der Wirkstoff wird hierbei molekular durch Diffusion aus dem Gas an die Phasengrenze überführt. Der Feststoff bildet sich dann erst an der Phasengrenze und gelangt, von dort unmittelbar in die Flüssigkeit, wo er sofort, vorzugsweise durch Schutzkolloide, stabilisiert wird, sodaß eine Agglomeration ausgeschlossen ist. Diese erste Dispersion wird unter Ausbildung einer zweiten Dispersion aus wirkstoffbeladener Flüssigkeit und fluidem Gas durch eine Verweilzeitstrecke geleitet. Die zweite Dispersion wird sodann durch Phasentrennung ohne eine vorherige Entspannung in eine Gasphase und eine dritte Dispersion aus Flüssigkeit und Wirkstoff getrennt. Ein Teil der dritten Dispersion wird schließlich zur Ausbildung der ersten Dispersion mit dem fluiden Gas wiederverwendet.

Vorteilhaft ist, daß ein großer Teil des Gases ohne erneute Verdichtung zur Auflösung des Wirkstoffs wiederverwendet werden kann.

Bevorzugt ist die Rückführung des abgetrennten Gases, das als fluides Gas zur Lösung des Wirkstoffes wiederverwendet wird. Vorzugsweise besteht der verwendete Wirkstoff aus einer Mischung aus mehreren Wirkstoffen. Bevorzugte Wirkstoffe sind Farbstoffe, Vitamine, Carotinoide, Polymere, Liposome, Pharmawirkstoffe oder Pflanzenschutzwirkstoffe. Bevorzugte fluide Gase sind CO₂, N₂O, Ethylen, Propan, H₂O, NH₃, Kohlenwasserstoffe, Alkohole und Mischungen aus diesen Substanzen. Vorzugsweise wird als Flüssigkeit Wasser oder ein organisches Lösungsmittel verwendet, das vorzugsweise mit Zusätzen von Schutzkolloiden wie Tensiden, Polymeren, Cellulosen, Dextrinen oder Proteinen wie Gelatine und/oder Emulgatoren oder ähnlichem angereichert ist.

Die Aufgabe der Erfindung wird auch durch die in den Ansprüchen beschriebene Vorrichtung gelöst. Eine Vorrichtung nach den Vorrichtungsansprüchen beinhaltet einen Extraktor für die Auflösung eines Wirkstoffes in einem fluiden Gas, der über eine Leitung mit einem Mischorgan für die Auflösung des fluiden wirkstoffbeladenen Gases in einer Flüssigkeit verbunden ist. Die Flüssigkeit wird dem Mischorgan über eine weitere Leitung zugeführt. Die Vorrichtung enthält darüber hinaus eine Verweilzeitstrecke, die mit einem Abscheider verbunden ist, in dem das Gas von der wirkstoffhaltigen Flüssigkeit getrennt wird. Der Abscheider weist Ableitungen für das abgetrennte Gas und für die abgetrennte Flüssigkeit auf. Außerdem enthält die Vorrichtung eine Rückführung für einen Teil der abgetrennten Flüssigkeit zum Mischorgan.

In einer bevorzugten Ausführungsform führt die Ableitung für das abgetrennte Gas dieses aus dem Abscheider zum Extraktor zurück.

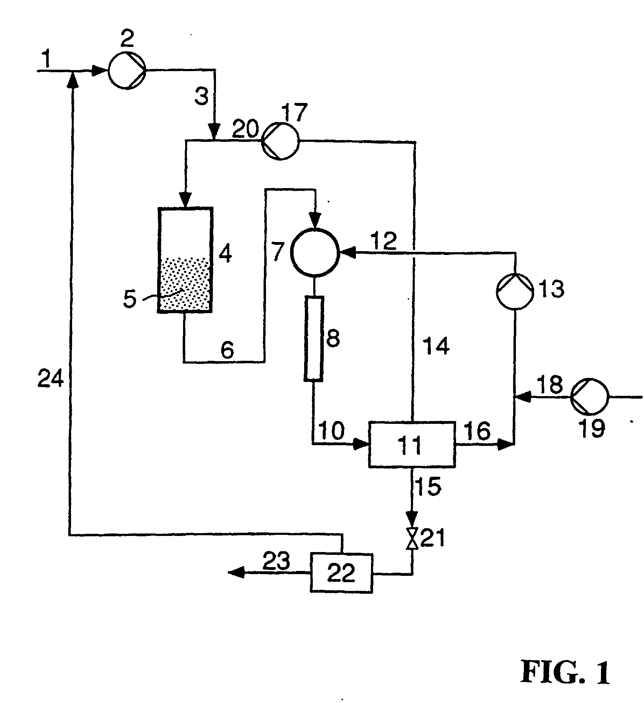

Die Erfindung wird im folgenden anhand der Figur 1 beschrieben. Es zeigt:
- Fig. 1:: Versuchsaufbau für eine Verfahrensvariante,

Bei der in Fig. 1 dargestellten Verfahrensvariante wird das fluide Gas ebenso dem Extraktor 4 mit dem Wirkstoff 5 zugeführt und erreicht als wirkstoffbeladenes fluides Gas das Mischorgan 7. In dem Mischorgan und der anschließenden Verweilzeitstrecke 8 erfolgt allerdings keine Lösung des fluiden Gases in der Flüssigkeit, sondern eine Dispersion des wirkstoffbeladenen fluiden Gases mit einer über Leitung 12 zugeführten Flüssigkeit, die mit dem selben Gas gesättigt ist. Durch Diffusion gelangt der im fluiden Gas gelöste Wirkstoff an die Phasengrenze, fällt dort in Form von Mikropartikeln aus und wird dann in der Flüssigkeit, vorzugsweise durch Schutzkolloide. Die nunmehr entstandene zweite Dispersion aus wirkstoffbeladener Flüssigkeit und fluidem Gas wird nicht entspannt, sondern direkt einem Abscheider 11 zugeführt. Über Ableitung 15, die mit einem Entspannungsventil 21 versehen ist, wird ein Teil der wirkstoffbeladenen Flüssigkeit entnommen. Diese Lösung wird im Abscheider 22 vom gelösten Gas getrennt und über Leitung 23 abgezogen. Das Gas wird über Leitung 24 wieder vor den Verdichter 2 zurückgeführt. Der andere Teil der wirkstoffbeladenen Flüssigkeit wird über Leitung 16 aus dem Abscheider abgezogen, durch Leitung 18 und Verdichter 19 mit frischer Flüssigkeit ergänzt und durch die Umwälzpumpe 13, die den Druckverlust in den Leitungen ausgleicht, im Kreis gefördert. Die Flüssigkeit wird dann über Leitung 12 wieder dem Mischorgan 7 zugeführt. Das abgetrennte fluide Gas wird über Leitung 14, Umwälzpumpe 17 und Leitung 20 zu dem Extraktor 4 zurückgeführt.

### Beispiel 1

In einem temperierbaren Autoklav mit 250 ml Inhalt wurden etwa 5 g β-Carotin vorgelegt. Ein N₂O-Strom (250 bar - 0,8 Kg pro Stunde) wurde auf 45°C vorgewärmt und in den Autoklav geleitet. Der mit Carotin beladene N₂O-Strom wurde dann durch eine Mischdüse in eine bei Athmosphärendruck mit 700 l pro Stunde geförderte wäßrige Lösung entspannt, die 3,8 Gew.% Gelatine enthielt. Die dabei entstandene Dispersion wurde anschließend in einem Abscheider in eine Gasphase und eine wirkstoffbeladene Flüssigkeit getrennt. Die Flüssigphase, die eine gelborange Farbe annahm, wurde zur Mischdüse zurückgeführt. Die Teilchengröße des darin dispergierten Carotins lag wiederum unter 1 µm.

## Patentansprüche

1. Verfahren zur Herstellung wirkstoffhaltiger flüssiger Dispersionen, bei dem ein Wirkstoff in einem fluiden Gas gelöst wird, **dadurch gekennzeichnet, daß** die erhaltene Lösung in einer mit diesem Gas gesättigten Flüssigkeit, die ein Nichtlöser für den Wirkstoff ist, unter Ausbildung einer ersten Dispersion aus Flüssigkeit und wirkstoffbeladenem fluidem Gas dispergiert wird,
die erste Dispersion durch eine Verweilzeitstrecke unter Ausbildung einer zweiten Dispersion aus wirkstoffbeladener Flüssigkeit und fluidem Gas geleitet wird,
die zweite Dispersion durch Entspannen in eine Gasphase und eine dritte Dispersion aus Flüssigkeit und Wirkstoff getrennt wird, und die dritte Dispersion zum Teil zur Ausbildung der ersten Dispersion mit dem fluiden Gas wiederverwendet wird.

2. Verfahren nach Anspruch 1, **dadurch gekennzeichnet, daß** das abgetrennte Gas zurückgeführt und als fluides Gas zur Lösung des Wirkstoffs wiederverwendet wird.

3. Verfahren nach einem der vorhergehenden Ansprüche, **dadurch gekennzeichnet, daß** der Wirkstoff aus einer Mischung aus mehreren Wirkstoffen besteht.

4. Verfahren nach einem der vorhergehenden Ansprüche, **dadurch gekennzeichnet, daß** als Wirkstoff ein Farbstoff, ein Vitamin, ein Carotinoid, ein Polymeres, ein Liposom, ein Pharmawirkstoff oder ein Pflanzenschutzwirkstoff, insbesondere ein Protein oder Peptid eingesetzt wird.

5. Verfahren nach einem der vorhergehenden Ansprüche, **dadurch gekennzeichnet, daß** als fluides Gas CO₂, N₂O, Ethylen, Propan, H₂O, NH₃, ein Kohlenwasserstoff, ein Alkohol oder eine Mischung aus diesen Substanzen eingesetzt wird.

6. Verfahren nach einem der vorhergehenden Ansprüche, **dadurch gekennzeichnet, daß** als Flüssigkeit Wasser oder ein organisches Lösungsmittel, vorzugsweise mit Zusätzen von Tensiden, Polymeren, Cellulosen, Dextrinen oder Proteinen wie Gelatine und/oder Emulgatoren eingesetzt wird.

7. Vorrichtung zur Durchführung des Verfahrens nach einem der Ansprüche 1 bis 6, **gekennzeichnet durch**
einen Extraktor (4) für die Auflösung eines Wirkstoffes (5) in einem fluiden Gas, ein mit dem Extraktor (4) über eine Leitung (6) verbundenes Mischorgan (7) für die Dispersion des fluiden wirkstoffbeladenen Gases in einer Flüssigkeit, die mit dem selben Gas gesättigt ist und die über eine Leitung (12) dem Mischorgan zugeführt wird,
eine Verweilzeitstrecke (8), die mit einem Abscheider (11) verbunden ist, in dem das Gas von der wirkstoffhaltigen Flüssigkeit getrennt wird,
eine Ableitung (14) für das abgetrennte Gas, sowie eine Ableitung (15) mit einem Entspannngsventil (21) für die abgetrennte Flüssigkeit aus dem Abscheider, und eine Rückführung (16) für einen Teil der abgetrennten Flüssigkeit zum Mischorgan (7).

8. Vorrichtung nach Anspruch 7, **dadurch gekennzeichnet, daß** die Leitung (14) das abgetrennte Gas aus dem Abscheider (11) zum Extraktor (4) zurückführt.

## Claims

1. A process for preparing active ingredient-containing liquid dispersions, in which an active ingredient is dissolved in a fluid gas, wherein the resulting solution is dispersed in a liquid which is saturated with this gas and which is a nonsolvent for the active ingredient to form a first dispersion of liquid and active ingredient-loaded fluid gas, the first dispersion is passed through a holdup section to form a second dispersion of active ingredient-loaded liquid and fluid gas, the second dispersion is separated by decompression into a gas phase and a third dispersion of liquid and active ingredient, and the third dispersion is in part reused to form the first dispersion with the fluid gas.

2. A process as claimed in claim 1, wherein the gas which has been separated off is recycled and reused as fluid gas to dissolve the active ingredient.

3. A process as claimed in any of the preceding claims, wherein the active ingredient consists of a mixture of a plurality of active ingredients.

4. A process as claimed in any of the preceding claims, wherein a dye, a vitamin, a carotenoid, a polymer, a liposome, an active pharmaceutical ingredient or a crop protection agent, in particular a protein or peptide, is used as active ingredient.

5. A process as claimed in any of the preceding claims, wherein CO₂, N₂O, ethylene, propane, H₂O, NH₃, a hydrocarbon, an alcohol or a mixture of these substances is used as fluid gas.

6. A process as claimed in any of the preceding claims, wherein water or an organic solvent, preferably with additions of surfactants, polymers, celluloses, dextrins or proteins such as gelatin and/or emulsifiers is used as liquid.

7. An apparatus for carrying out the process as claimed in any of claims 1 to 6, which comprises an extractor (4) to dissolve an active ingredient (5) in a fluid gas, a mixer (7) which is connected via a line (6) to the extractor (4) to disperse the fluid gas loaded with active ingredient in a liquid, which is saturated with the same gas and which is fed to the mixer via a line (12), a holdup section (8) which is connected to a separator (11) in which the gas is separated from the liquid containing active ingredient, a discharge line (14) for the gas which has been separated off, and a discharge line (15) with a decompression valve (21) for the liquid which has been separated off from the separator, and a return line (16) for part of the liquid which has been separated off to the mixer (7).

8. An apparatus as claimed in claim 7, wherein the line (14) returns the gas which has been separated off from the separator (11) to the extractor (4).

## Revendications

1. Procédé de préparation de dispersions liquides ayant une teneur en substance active, dans lequel une substance active est dissoute dans un gaz fluide, **caractérisé en ce que** la solution obtenue est dispersée dans un liquide qui est saturé de ce gaz et qui n'est pas solvant pour la substance active, avec formation d'une première dispersion à base du liquide et du gaz fluide chargé de substance active, **en ce que** la première dispersion est conduite au travers d'une section de temps de séjour, avec formation d'une deuxième dispersion à base de liquide chargé de substance active et de gaz fluide, **en ce que** la deuxième dispersion est séparée par détente en une phase gazeuse et une troisième dispersion à base de liquide et de substance active, et **en ce que** la troisième dispersion est partiellement réutilisée pour la formation de la première dispersion avec le gaz fluide.

2. Procédé suivant la revendication 1, **caractérisé en ce que** le gaz isolé est recyclé et réutilisé comme gaz fluide pour la dissolution de la substance active.

3. Procédé suivant l'une des revendications précédentes, **caractérisé en ce que** la substance active est constituée d'un mélange de plusieurs substances actives.

4. Procédé suivant l'une des revendications précédentes, **caractérisé en ce que**, comme substance active, on met en oeuvre un colorant, une vitamine, un carotinoïde, un polymère, un liposome, une substance active pharmaceutique ou une substance active phytosanitaire, en particulier une protéine ou un peptide.

5. Procédé suivant l'une des revendications précédentes, **caractérisé en ce que**, comme gaz fluide, on met en oeuvre du CO₂, du N₂O, de l'éthylène, du propane, du H₂O, du NH₃, un hydrocarbure, un alcool ou un mélange de ces substances.

6. Procédé suivant l'une des revendications précédentes, **caractérisé en ce que**, comme liquide, on met en oeuvre de l'eau ou un solvant organique, de préférence avec des additions d'agents tensioactifs, de polymères, de celluloses, de dextrines ou de protéines, comme de la gélatine et/ou d'agents émulsionnants.

7. Dispositif pour la mise en oeuvre du procédé suivant l'une des revendications 1 à 6, **caractérisé par**
un extracteur (4) pour la dissolution d'une substance active (5) dans un gaz fluide,
un organe de mélange (7) relié à l'extracteur (4) par un conduit (6) et destiné à la dispersion du gaz fluide chargé de substance active dans un liquide, qui est saturé du même gaz et qui est amené à l'organe de mélange par l'intermédiaire d'un conduit (12),
une section de temps de séjour (8), qui est reliée à un séparateur (11) dans lequel le gaz est séparé du liquide contenant de la substance active,
une dérivation (14) pour le gaz isolé, ainsi qu'une dérivation (15) munie d'une soupape de détente (21) pour le liquide isolé dans le séparateur, et
un recyclage (16) pour une partie du liquide isolé vers l'organe de mélange (7).

8. Dispositif suivant la revendication 7, **caractérisé en ce que** le conduit (14) recycle le gaz isolé du séparateur (11) en direction de l'extracteur (4).
